# EUROPEAN PATENT APPLICATION

(11) **EP 1 637 868 A1**
(43) Date of publication of application: **22.03.2006**
(21) Application number: 04745872.4
(22) Date of filing: 14.06.2004
(51) Int. Cl.: G01N 21/17, G01N 33/20

(54) **IMPURITY MEASURING METHOD AND DEVICE**

(30) Priority: 12.06.2003 JP 2003167310
(71) Applicant: Nippon Light Metal Company Ltd., Shinagawa-ku, Tokyo 140-8628 (JP); Toyota Jidosha Kabushiki Kaisha, Toyota-shi, Aichi 471-8571 (JP)
(72) Inventor: KURAMASU, Yukio, Light Metal Company, Ltd., Ihara-gun, Shizuoka 4213203 (JP); NUKAMI, Tetsuya, Toyota-shi, Aichi 4718571 (JP)
(74) Representative: Maser, Jochen
(86) International application number: PCT/JP2004/008318
(87) International publication number: WO 2004/111619

(57) **Abstract**

An impurity measuring device includes a table (T) on which a sample (S) is to be placed with its fracture surface (h) facing up, an illuminating means (7) for irradiating the fracture surface (h) with light (L) from a plurality of directions, an image sensing means for sensing an image of the fracture surface (h) irradiated with the light (L), continuous tone color image processing means for processing the sensed image into a continuous tone color image, and a binarizing means for binarizing the continuous tone color image through comparison between the result of the continuous tone color image processing and a threshold value. As the fracture surface (h) is irradiated with the light (L) from the plurality of directions, the image obtained by sensing the image of the fracture surface (h) is free from shading or optical irregularities caused by minute irregularities on the fracture surface (h). Therefore, impurities in the sample (S) can be accurately detected from the fracture surface (h) by subjecting the image to the continuous tone color image processing and binarization.

## Description

### Technical Field

The present invention relates to an impurity measuring method and device and, more particularly, to a method and apparatus which can measure impurities in real time easily at, e.g., a foundry.

### Background Art

An aluminum alloy contains non-metallic inclusions, unnecessary metal elements, segregated structures of a specific metal element, or the like as impurities. For example, the non-metallic inclusions are locations where a fracture starts to occur in a cast aluminum alloy to decrease the strength and elongation. Therefore, before a casting process, molten aluminum is subjected to a residual removing process or standing process by using a flux to remove the non-metallic inclusions.

In general, when a metal structure of aluminum or the like is to be observed, its sample is mirror-polished and subjected to a corrosion process, and is then observed with an optical microscope or the like. According to this observation method, as the sample is mirror-polished, the surface to be observed has no steps. With the observation method of subjecting the sample to mirror polishing and the corrosion process, however, cumbersome preparatory operation is required in advance, and the metal structure of, e.g., aluminum or the like which should be cast in the casting process cannot be observed easily and quickly.

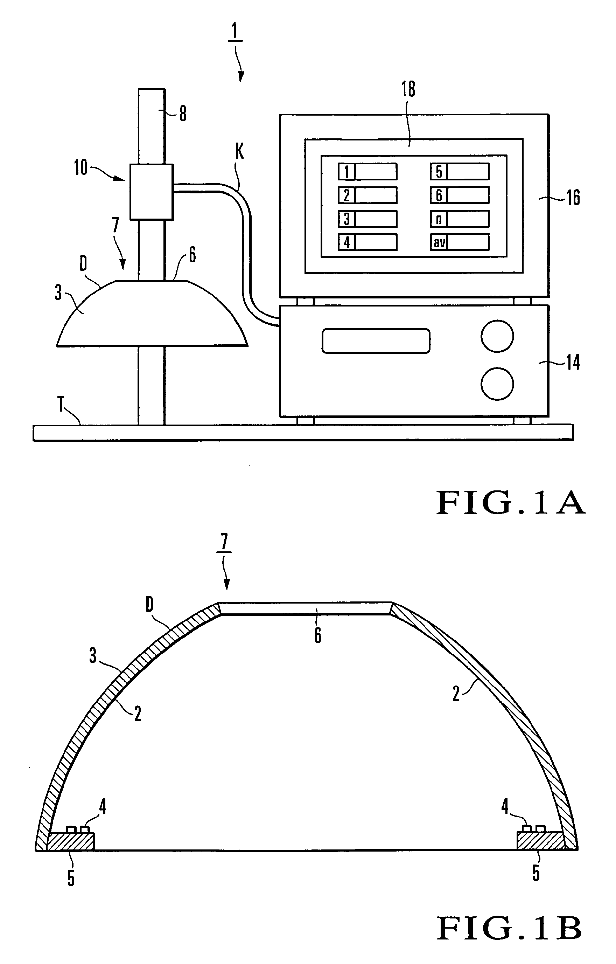

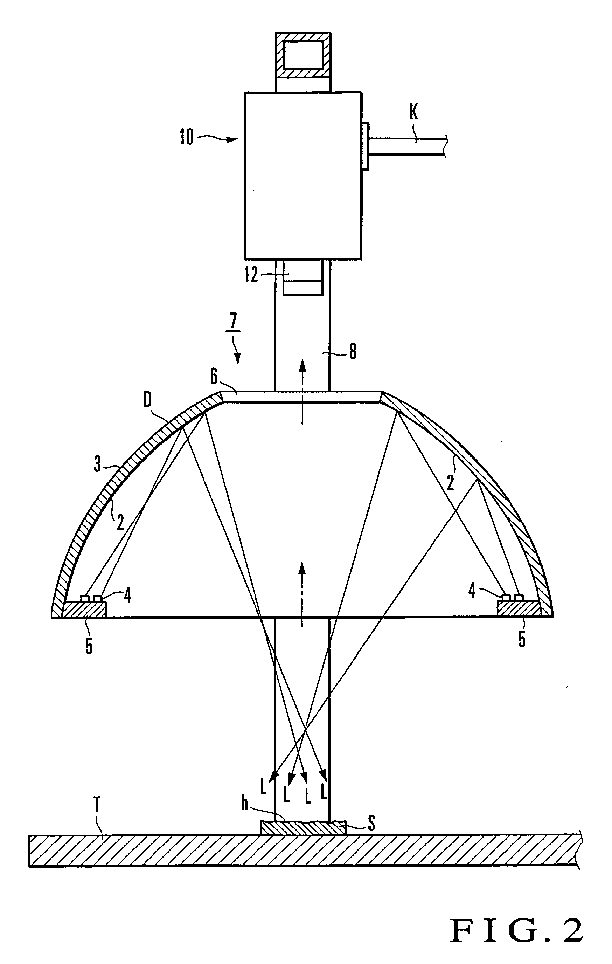

In order to solve these problems, a so-called K-mold method has been employed as a method of removing non-metallic inclusions from molten aluminum and measuring the residual amount of the non-metallic inclusions in the molten metal at the foundry simply and preliminarily. According to the K-mold method, part of molten aluminum is extracted and cast in a casting mold having a small-height rectangular parallelepiped cavity. The obtained sample formed of a plate-like rectangular parallelepiped cast piece is broken along its widthwise direction. The obtained fracture surface is observed with the naked eye or optical microscope to measure the total number of non-metallic inclusions (for example, see patent reference 1).
Patent Reference 1: Japanese Utility Model Publication No. 52-17449 (Pages 1 and 2, Figs. 1 and 2).

### Disclosure of Invention

### Problems to be Solved by the Invention

With the K-mold method, however, as the fracture surface of the sample has irregularities, when it is directly illuminated with light, shading or optical irregularities occur, and measurement of the non-metallic inclusions tends to become unstable. In addition, since the operator performs the measurement with the naked eye, variations tend to occur due to individual difference to lack reliability.

The above problems commonly arise not only in measurement of the non-metallic inclusions but also in measurement of impurities such as unnecessary metal elements, segregated structures of a specific metal element, or the like.

### Means of Solution to the Problems

The present invention has been made to solve the above problems, and has as its object to enable detection of impurities in a sample from the fracture surface accurately.

In order to achieve the above object, according to the present invention, there is provided an impurity measuring method characterized by comprising the steps of arranging a sample having a fracture surface on a table with the fracture surface facing up, irradiating the fracture surface with light from a plurality of directions from above the table, sensing an image of the fracture surface irradiated with the light, processing the sensed image into a continuous tone color image, and binarizing the continuous tone color image through comparison between a result of the continuous tone color image processing and a threshold value.

According to the present invention, there is also provided an impurity measuring device characterized by comprising a table on which a sample having a fracture surface facing up, illuminating means, arranged above the table, for irradiating the fracture surface with light from a plurality of directions, image sensing means for sensing an image of the fracture surface irradiated with the light, continuous tone color image processing means for processing the sensed image into a continuous tone color image, and binarizing means for binarizing the continuous tone color image through comparison between a result of the continuous tone color image processing and a threshold value.

### Effect of the Invention

According to the present invention, as the fracture surface of the sample is irradiated with light from a plurality of directions, the image obtained by sensing the image of the fracture surface is free from shading or optical irregularities caused by minute irregularities on the fracture surface. When the image is subjected to continuous tone color image processing and binarization, impurities in the sample can be accurately detected from the fracture surface.

### Brief Description of Drawings

[Fig. 1A] Fig. 1A is a front view showing the overall structure of an impurity measuring device according to an embodiment of the present invention.
[Fig. 1B] Fig. 1B is a vertical sectional view showing the structure of a reflection dome.
[Fig. 2] Fig. 2 is a view showing the relationship among a sample on a table, the reflection dome, and a CCD camera.
[Fig. 3] Fig. 3 is a view showing the configuration of a computer.
[Fig. 4] Fig. 4 is a block diagram showing a functional portion realized by a CPU.
[Fig. 5] Fig. 5 is a flowchart showing the flow of an impurity measuring device according to the embodiment of the present invention.

### Best Mode for Carrying Out the Invention

An embodiment of the present invention will be described in detail with reference to the accompanying drawings.

As shown in Fig. 1A, an impurity measuring device 1 has a table T on which a sample S (not shown) is to be arranged. The table T suffices as far as it has at least a flat surface.

According to this embodiment, the sample S will be exemplified by an aluminum sample S. Note that in the following description, what is merely described as "aluminum" includes an aluminum alloy as well. For example, the aluminum sample S is obtained by extracting part of molten aluminum immediately before semicontinuous casting, casting the extracted molten aluminum by, e.g., a casting mold for the K-mold method, and dividing the obtained plate-like rectangular parallelepiped cast piece by breaking it at a plurality of positions along its widthwise direction. The sample S is placed on the surface of the table T with its fracture surface h facing up.

An illuminating unit 7 is arranged above the table T to irradiate the fracture surface h of the sample S with light from a plurality of directions. The illuminating unit 7 includes light-emitting diodes (light sources) 4 which emit light and a reflection dome (reflection member) D which reflects the light from the light-emitting diodes (light sources) 4.

As shown in Fig. 1B, the reflection dome D has an outer surface 3 having a substantially semicircular section and a concave reflection surface 2 which has a shape similar to the reflection dome D (that is, having a substantially semicircular section) and opens downward. The concave reflection surface 2 is a mirror surface which is curved with a predetermined curvature. The concave reflection surface 2 may have minute irregularities to scatter the light.

A ring 5 is attached along the inner edge of the concave reflection surface 2. A large number of light-emitting diodes (LEDs) 4 are arranged on the ring 5 in a ring shape to project upward in two, inner and outer rows. As the light-emitting diodes 4, for example, those which are made of Ga-P doped with oxygen and nitrogen to emit red light and green light, those which are made of Ga-As to emit infrared light, or those which emit blue light are used. The light-emitting diodes 4 are comparatively compact. Thus, the light-emitting diodes 4 can be attached to the inner edge of the concave reflection surface 2 of the reflection dome D compactly. Moreover, when the high-luminance, high-directivity light emitted from the light-emitting diodes 4 is reflected by the concave reflection surface 2, it can be prevented from being shielded by the light sources.

An opening 6 which is quadrangular (square or rectangular) or circular when seen from the top is formed in the vicinity of the vertex of the reflection dome D. As shown in Fig. 2, a CCD camera (imaging means) 10 is arranged above the opening 6 of the reflection dome D. A light-incident cylinder 12 incorporating the optical lens of the CCD camera 10 is directed to the fracture surface h of the sample S, arranged on the surface of the table T, through the opening 6.

The reflection dome D is attached to a support column 8 standing upward from the table T with a metal fixture (not shown) to be vertically movable. Above the reflection dome D, the CCD camera 10 is attached to the same support column 8 to be vertically movable.

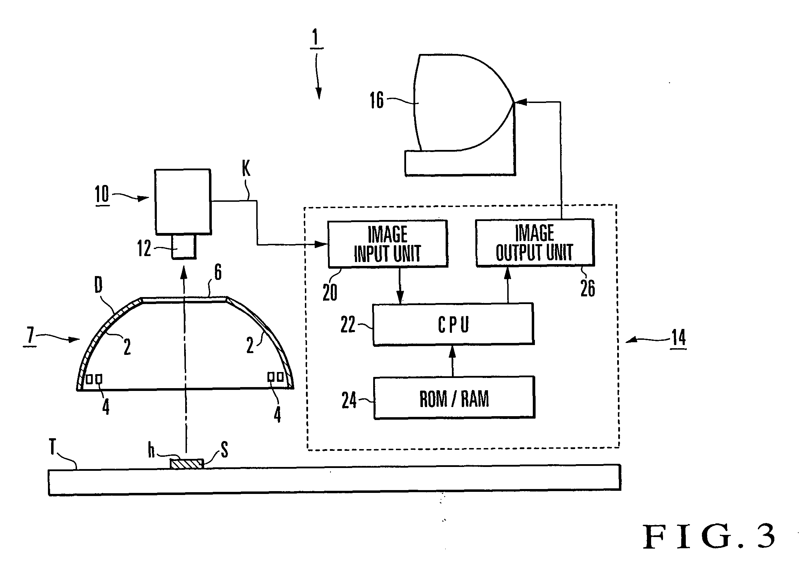

As shown in Fig. 1A, a cable K extending from the CCD camera 10 is connected to a personal computer (arithmetic means) 14. As shown in Fig. 3, the computer 14 has an image input unit (interface) 20, central processing element (CPU) 22, storage (ROM/RAM) 24, and image output unit (interface) 26.

The image input unit 20 receives an image signal which is transmitted from the CCD camera 10 through the cable K.

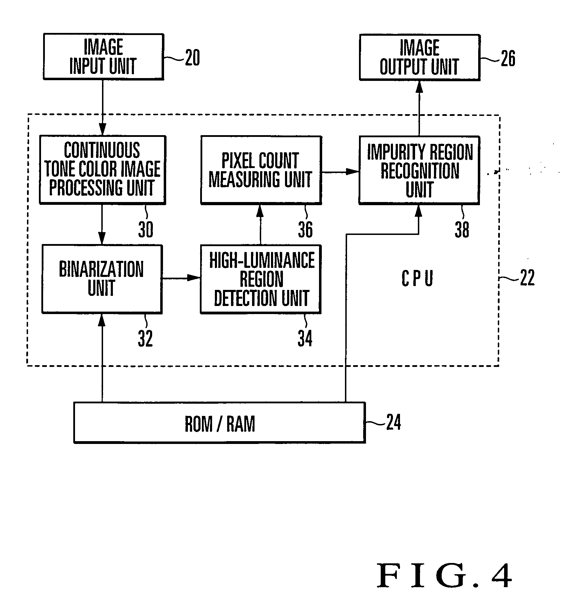

The central processing element 22 operates in accordance with a program to realize a continuous tone color image processing unit 30, binarization unit 32, high-luminance region detection unit 34, pixel count measurement unit 36, and impurity region recognition unit 38 shown in Fig. 4. The continuous tone color image processing unit 30 subjects an image input from the image input unit 20 to continuous tone color image processing. The binarization unit 32 subjects the image to binarization through comparison between the processing result of the continuous tone color image processing unit 30 and a luminance threshold value. The high-luminance region detection unit 34 detects an image region having a luminance higher than the threshold value from the image processed by the binarization unit 32. The pixel count measurement unit 36 measures the number of pixels of the image region detected by the high-luminance region detection unit 34. When the number of pixels measured by the pixel count measurement unit 36 is larger than a predetermined pixel count, the impurity region recognition unit 38 recognizes the image region detected by the high-luminance region detection unit 34 as a non-metallic inclusion region. When the measured number of pixels is smaller than the predetermined pixel count, the impurity region recognition unit 38 does not recognize the detected image region as a non-metallic inclusion region.

The storage 24 stores data such as the luminance threshold value, predetermined pixel count, and the like described above. Thus, in the process of the central processing element 22, data stored in the storage 24 is sequentially read out when necessary. The program which controls the operation of the central processing element 22 is also stored in the storage 24.

The processing result of the central processing element 22 is displayed on a display 18 of a monitor 16 through the image output unit 26, as shown in Figs. 1A and 3, and printed by a printer (not shown) when necessary.

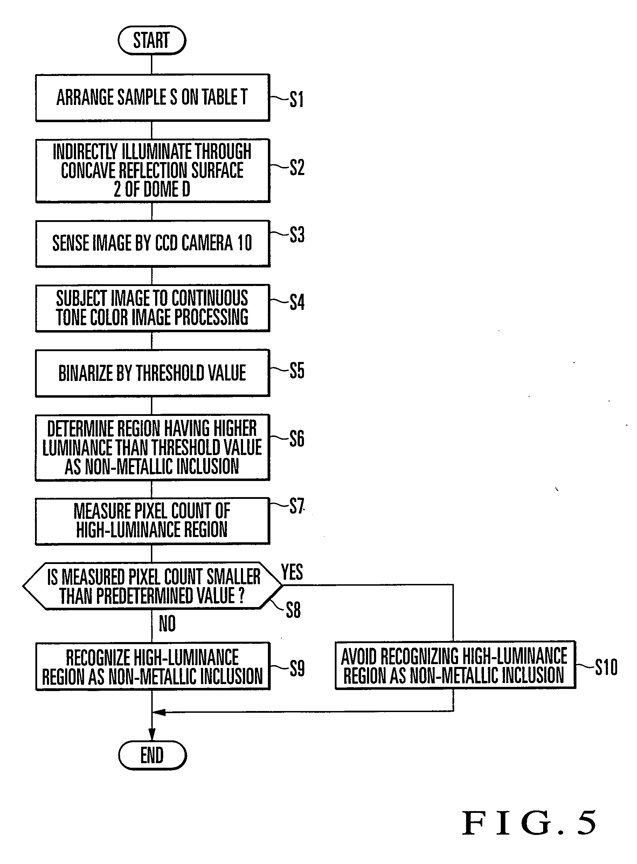

A method of measuring a non-metallic inclusion in aluminum by using the impurity measuring device 1 will be described with reference to Fig. 5.

As shown in Fig. 2, the aluminum sample S to be measured is arranged at a predetermined position on the surface of the table T with its fracture surface h facing up (step S1). The sample S is obtained by casting part of molten aluminum held at about 700°C by a casting mold for the K-molding method and breaking the obtained plate-shaped cast piece.

Subsequently, light L emitted from a large number of light-emitting diodes 4 is reflected by the concave reflection surface 2 of the reflection dome D, as indicated by arrows of solid lines in Fig. 2. The light L which is reflected in a plurality of random directions irradiates the fracture surface h of the sample S as indirect illumination (step S2). At this time, as the fracture surface h is irradiated with the light L from the plurality of random directions, occurrence of shading, optical irregularities, halation, and the like which are caused by the minute irregularities on the fracture surface h can be prevented.

In this state, the image of the fracture surface h of the sample S is sensed by the charge-coupled devices in the CCD camera 10 from the light-incident cylinder 12 through the opening 6 of the reflection dome D, as indicated by arrows of alternate long and short dashed lines in Fig. 2 (step S3). The obtained image signal is transmitted from the image input unit 20 to the central processing element 22 of the computer 14 through the cable K.

The central processing element 22 first subjects the image of the fracture surface h to continuous tone color image processing (step S4). More specifically, the tone of each pixel of the image of the fracture surface h is converted into a gray scale value in the form of an 8-bit density value ranging from, e.g., white = 0 to back = 255.

Subsequently, the image is subjected to the binarization (step S5). More specifically, the luminance threshold value (threshold value) is read out from the storage 24 in advance. The luminances of the respective pixels obtained by the continuous tone color image processing are compared with the threshold value and sorted into a high-luminance group and low-luminance group. The threshold value is a value which is preset in accordance with the type of the material (aluminum in this embodiment) of the sample S.

Then, an image region having a higher luminance than the luminance threshold value is detected from the image, and the detected region is determined as a non-metallic inclusion region (step S6). The number of pixels of the detected image region is measured (step S7) .

A predetermined minimal pixel count, which is defined as the minimal pixel count when a non-metallic inclusion is present in the aluminum sample S, is compared with the pixel count measured in step S7. If the measured pixel count is larger than the predetermined minimal pixel count (No in step S8), the image region detected in step S6 is recognized as a non-metallic inclusion region (step S9). In contrast to this, if the measured pixel count is smaller than the predetermined minimal pixel count (Yes in step S8), determination of step S6 is corrected so the image region in question will not be recognized as a non-metallic inclusion region (step S10). When the number of pixels of the entire image is 240,000, the predetermined minimal pixel count is on the order of several 10. The minimal pixel count may be read out from the storage 24 when necessary.

In this manner, according to this embodiment, even when an image region is once determined as a non-metallic inclusion from its luminance, if the number of pixels of this image region is smaller than the minimal pixel count of the non-metallic inclusion formed in aluminum, the determination as being a non-metallic inclusion is corrected and this region is not recognized as a non-metallic inclusion. Thus, an error in determination of optical analysis can be eliminated reliably.

With the above process, the absence/presence of non-metallic inclusions in the fracture surface h which is the source of the image, and the total number of the non-metallic inclusions can be measured accurately and quickly, and this measurement can be operated easily at the foundry as well.

In step S6, it suffices as far as an image region having a higher luminance than the luminance threshold value is detected from the image. Therefore, this region need not always be determined as a non-metallic inclusion region.

The above steps S1 to S10 can be performed sequentially and continuously for a plurality of fracture surfaces h of the sample S. Hence, as shown in Fig. 1A, the total number of non-metallic inclusions of the images (1 to n) sensed for the respective fracture surfaces h and an average value (av) of the non-metallic inclusions in the entire images can be measured and monitored on the display 18 of the monitor 16.

If the total number of non-metallic inclusions of the plurality of measured fracture surfaces h and the average value of the entire non-metallic inclusions fall within allowable ranges for aluminum, the molten aluminum may be directly cast into the casting mold of a semicontinuous casting apparatus (not shown), so that a cast material such as an aluminum slab or billet which has a necessary purity or alloy component can be obtained reliably with no loss.

In contrast to this, if the total number of non-metallic inclusions of the plurality of measured fracture surfaces h and the average value of the entire non-metallic inclusions fall outside the allowable ranges, the molten aluminum is not subjected to semicontinuous casting but is sent to a known aluminum refining process to remove non-metallic inclusions. After that, the measurement method described above is performed again for the sample which has been partly extracted.

Therefore, according to the method of measuring a non-metallic inclusion in aluminum using the impurity measuring device 1, molten aluminum can be formed into respective types of cast materials stably with no loss, thus contributing to a decrease in cost of the casting process.

The present invention is not limited to the embodiment described above.

The sample S is not limited to aluminum. A sample made of steel, cast iron, cast steel, various types of special steels, stainless steel, titanium and a titanium alloy, copper and a copper alloy, zinc and a zinc alloy, Ni and a Ni alloy, Mg and a Mg alloy, Su and a Su alloy, or lead and a lead alloy can also be subjected to measurement.

The impurities as the measurement target are not limited to non-metallic inclusions, but also include crystals of unnecessary metal elements, segregated structures of a specific metal element, and the like.

Alternatively, a slide holder having a plurality of recesses equidistantly may be arranged on the table T. Samples S may be individually inserted in the plurality of recesses of the holder with their fracture surfaces h facing up. The holder may be moved manually or automatically moved along a guide rail (not shown) to sequentially image the respective fracture surfaces h.

The binarization which is performed after continuous tone color image processing can employ not only the luminance threshold value but also a lightness threshold value or density threshold value.

It is also possible to determine an image region having a luminance or the like higher or lower than the threshold value as a segregated portion in an aluminum alloy or the like, or a crystal of an unnecessary metal element.

The position of the opening 6 of the reflection dome D is not limited to the vicinity of the vertex of the reflection dome D, but the opening 6 may be formed at an arbitrary position of the reflection dome D. In this case, the CCD camera 10 is arranged at a position from where the fracture surface h of the sample S can be seen through the opening 6. Accordingly, the position of the CCD camera 10 is not limited to above the opening 6, but sometimes the CCD camera 10 may be arranged obliquely above the opening 6.

The positions of the table T, reflection dome D, CCD camera 10, and the like of the impurity measuring device 1 shown in Fig. 1A are relative. As far as the sample S can be arranged on the table T as it is supported by a clip or holder, the table T, reflection dome D, CCD camera 10, and the like can be set at arbitrary inclinations.

As the image sensing means, other than a CCD (charge couple device) camera including a digital camera, for example, a video camera can also be used.

The computer 14 and monitor 16 need not be arranged on the table T but may be arranged at other positions.

The arithmetic means is not limited to the computer 14. A control device such as a controller which exhibits the similar function can be used as the arithmetic means.

The impurity measuring method and device according to the present invention can be appropriately changed within a range not departing from the spirit of the invention.

### Industrial Applicability

As described above, the impurity measuring method and device according to the present invention are effective for measuring non-metallic inclusions, crystal of unnecessary metal elements, segregated structures of a specific metal element, or the like which are contained in a metal or the like.

## Claims

1. An impurity measuring method **characterized by** comprising the steps of:
arranging a sample having a fracture surface on a table with the fracture surface facing up;
irradiating the fracture surface with light from a plurality of directions from above the table;
sensing an image of the fracture surface irradiated with the light;
processing the sensed image into a continuous tone color image; and
binarizing the continuous tone color image through comparison between a result of the continuous tone color image processing and a threshold value.

2. An impurity measuring method according to claim 1, **characterized in that** the step of irradiating with the light includes the step of irradiating the fracture surface with indirect illumination.

3. An impurity measuring method according to claim 1, **characterized in that** the step of irradiating with the light includes the step of irradiating the fracture surface with indirect illumination of light from a light source which is reflected by a concave reflection surface having a substantially semicircular section.

4. An impurity measuring method according to claim 1, **characterized by** further comprising the steps of:
detecting an image region having a higher luminance than the threshold value from the binarized image; and
measuring a pixels count of the detected image region.

5. An impurity measuring method according to claim 4, **characterized by** further comprising the steps of:
recognizing the detected image region as an impurity region when the measured pixel count is larger than a predetermined pixel count; and
avoiding recognizing the detected image region as an impurity region when the measured pixel count is smaller than the predetermined pixel count.

6. An impurity measuring method according to claim 1, **characterized in that**
the step of arranging a sample includes the step of arranging an aluminum sample on the table.

7. An impurity measuring method according to claim 1, **characterized in that** the step of sensing an image includes the step of sensing an image of the fracture surface by a CCD camera.

8. An impurity measuring device **characterized by** comprising:
a table on which a sample having a fracture surface facing up;
illuminating means, arranged above the table, for irradiating the fracture surface with light from a plurality of directions;
image sensing means for sensing an image of the fracture surface irradiated with the light;
continuous tone color image processing means for processing the sensed image into a continuous tone color image; and
binarizing means for binarizing the continuous tone color image through comparison between a result of the continuous tone color image processing and a threshold value.

9. An impurity measuring device according to claim 8, **characterized in that** said illuminating means includes
a light source which emits light, and
a reflection member which reflects the light from said light source.

10. An impurity measuring device according to claim 9, **characterized in that**
said reflection member comprises a reflection dome which has a substantially semicircular section and a downward concave reflection surface, and
said light source comprises a plurality of light sources which are arranged to face upward along an inner edge of said concave reflection surface of said reflection dome.

11. An impurity measuring device according to claim 10, **characterized in that** said light sources comprise light-emitting diodes.

12. An impurity measuring device according to claim 10, **characterized in that**
said reflection dome has an opening in the vicinity of a vertex thereof, and
said image sensing means is arranged above the opening.

13. An impurity measuring device according to claim 8, **characterized by** further comprising:
high-luminance region detection means for detecting an image region having a higher luminance than the threshold value from the image binarized by said binarizing means; and
pixel count measuring means for measuring a pixel count of the image region detected by said high-luminance region detection means.

14. An impurity measuring device according to claim 13, **characterized by** further comprising impurity region recognizing means for recognizing the image region detected by said high-luminance region detection means as an impurity region when the pixel count measured by said pixel count measuring means is larger than a predetermined pixel count, and avoiding recognizing the detected image region as an impurity region when the measured pixel count is smaller than the predetermined pixel count.

15. An impurity measuring device according to claim 8, **characterized in that** the sample comprises aluminum.

16. An impurity measuring device according to claim 8, **characterized in that** said image sensing means comprises a CCD camera.
